Europäisches Patentamt

**European Patent Office** (11) Numéro de publication: **0 064 002**

Office européen des brevets **A2**

(12) **DEMANDE DE BREVET EUROPEEN**

(21) Numéro de dépôt: **82400707.4**

(22) Date de dépôt: **20.04.82**

(51) Int. Cl.³: **A 61 N 1/36**

(30) Priorité: **23.04.81 FR 8108092**

(43) Date de publication de la demande: **03.11.82**
**Bulletin 82/44**

(84) Etats contractants désignés: **CH DE GB IT LI NL**

(71) Demandeur: **ELA MEDICAL, Société Anonyme dite:,
98 rue Maurice Arnoux, F-92120 Montrouge (FR)**

(72) Inventeur: **Ripart, Alain, 17, rue de la Gruerie, F-91190 Gif
Sur Yvette (FR)**
Inventeur: **Jacobson, Peter Michael, 28, rue Louis
Morard, F-75014 Paris (FR)**

(74) Mandataire: **Derambure, Christian, Cabinet BUGNION
ASSOCIES SARL 116, boulevard Haussmann,
F-75008 Paris (FR)**

(54) **Procédé de commande et de réglage d'un appareil tel qu'un stimulateur cardiaque.**

(57) L'invention concerne un procédé de commande et de réglage d'un appareil tel qu'un stimulateur cardiaque.

Dans le procédé on mesure la résistance interne de la batterie d'alimentation de l'appareil dans un premier mode opératoire dans lequel on ramène le rythme du stimulateur au rythme magnétique, on fait recevoir par le stimulateur les informations de commande à partir du programmeur extérieur dans un second mode opératoire dans un troisième mode on fait recevoir par le stimulateur l'instruction de programmation d'au moins un paramètre opératoire; on envoie en retour au programmeur les informations de contrôle sur les valeurs programmées; on mesure le seuil de stimulation du patient; on fait passer le stimulateur dans l'un des modes opératoires normaux.

## Procédé de commande et de réglage d'un appareil tel qu'un stimulateur cardiaque.

L'invention concerne un procédé de commande et de réglage d'un appareil ou instrument médical, notamment un stimulateur cardiaque implantable.

On connaît déjà de tels dispositifs, mais ils ne sont pas totalement satisfaisants du fait des exigences toujours accrues des progrès de la médecine. Par exemple, ils ne peuvent programmer et contrôler un nombre suffisant de paramètres, ou déterminer le degré de décharge de la batterie, etc.

Le but de l'invention est de pallier ces inconvénients en proposant un procédé et un dispositif d'application de ce procédé.

Plus spécifiquement, le but de l'invention est de permettre de réaliser de façon simple, efficace et fiable, en premier lieu la programmation, depuis l'extérieur du corps du patient recevant le stimulateur, d'une série de paramètres de fonctionnement dudit stimulateur ; en deuxième lieu, d'une part la confirmation par télémétrie que ces paramètres ont été correctement reçus et d'autre part la mesure du rythme de pulsation et du rythme magnétique ; en troisième lieu la mesure de la résistance interne de la batterie pour déterminer sa fin de vie ; en quatrième lieu, l'ajustement automatique de l'impédence d'entrée du stimulateur à la valeur optimale, selon les phases opératoires du stimulateur ; en cinquième lieu, un test automatique du seuil des dépolari-

2

0064002

sations ; en sixième lieu de limiter le rythme à la valeur maximale de 120 impulsions par minute.

Le procédé est caractérisé par le fait qu'on mesure la résistance interne de la batterie d'alimentation comme indication d'un prochain épuisément de celle-ci dans un premier mode opératoire de commande FMR, dans lequel on ramène également le rythme du stimulateur à un rythme fixé à l'avance notamment le rythme magnétique ; on fait recevoir par le stimulateur les informations de commande à partir du programmeur extérieur dans un second mode opératoire de commande MAG à partir duquel le stimulateur peut passer à l'autres modes. Dans un troisième mode opératoire de commande PRG on fait recevoir par le stimulateur de la part du programmeur externe, par voie télémétrique, l'instruction de programmation d'au moins un paramètre opératoire, les données des mémoires du stimulateur étant modifiées pour correspondre à l'instruction programmée ; on envoit en retour au programmeur extérieur des informations de contrôle sur les valeurs programmées dans un quatrième mode opératoire de commande TEL ; on mesure le seuil de stimulation du patient dans un cinquième mode opératoire de commande TSL ; dans un sixième mode opératoire de commande VTL le stimulateur passe à l'un des modes opératoires normaux VOO, VVI ou VVT, le dernier programme.

Le procédé selon l'invention est plus spécialement réalisé par le dispositif proposé à un très haut degré de stabilité et une consommation d'énergie très faible du fait qu'une unique combinaison d'éléments est utilisée, avec un nombre minimal de composants, compacts et sûrs. Il est utilisé à cette fin un microprocesseur séquentiel programmé pour accomplir la majorité des fonctions logiques demandées.

L'invention proposée sera bien comprise d'après la description d'une forme de mise en service du procédé et d'une forme d'exécution du dispositif ainsi que les dessins annexés dont :

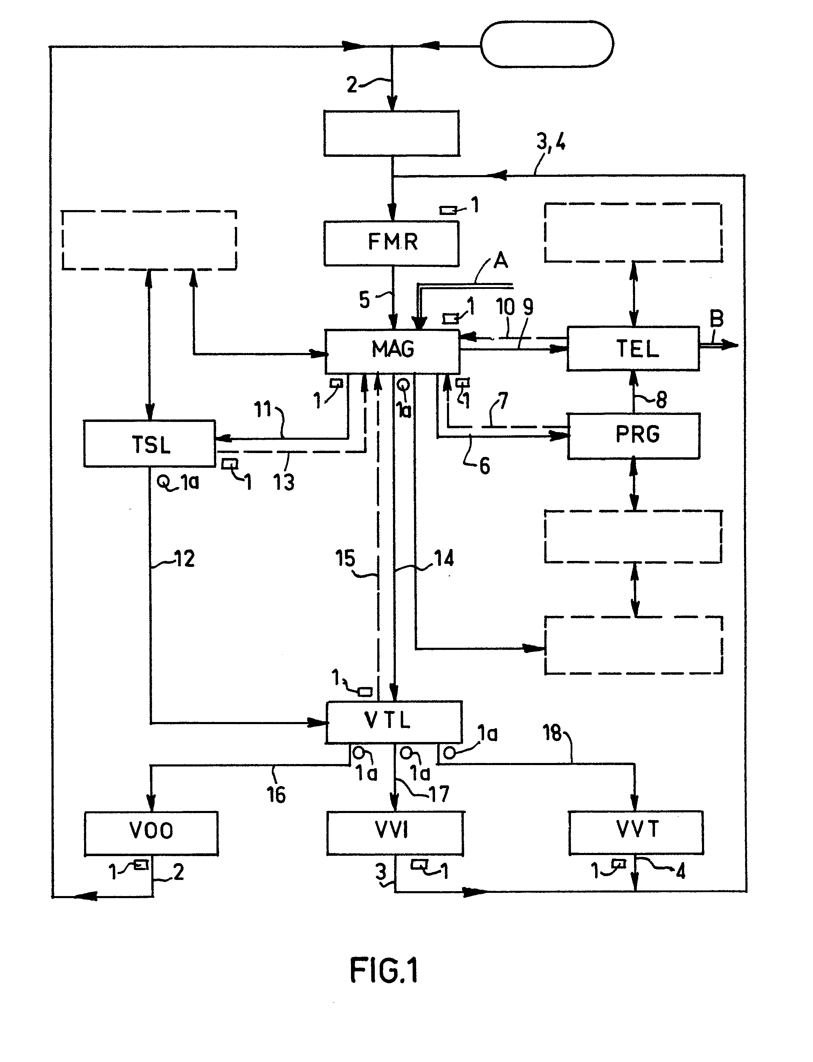

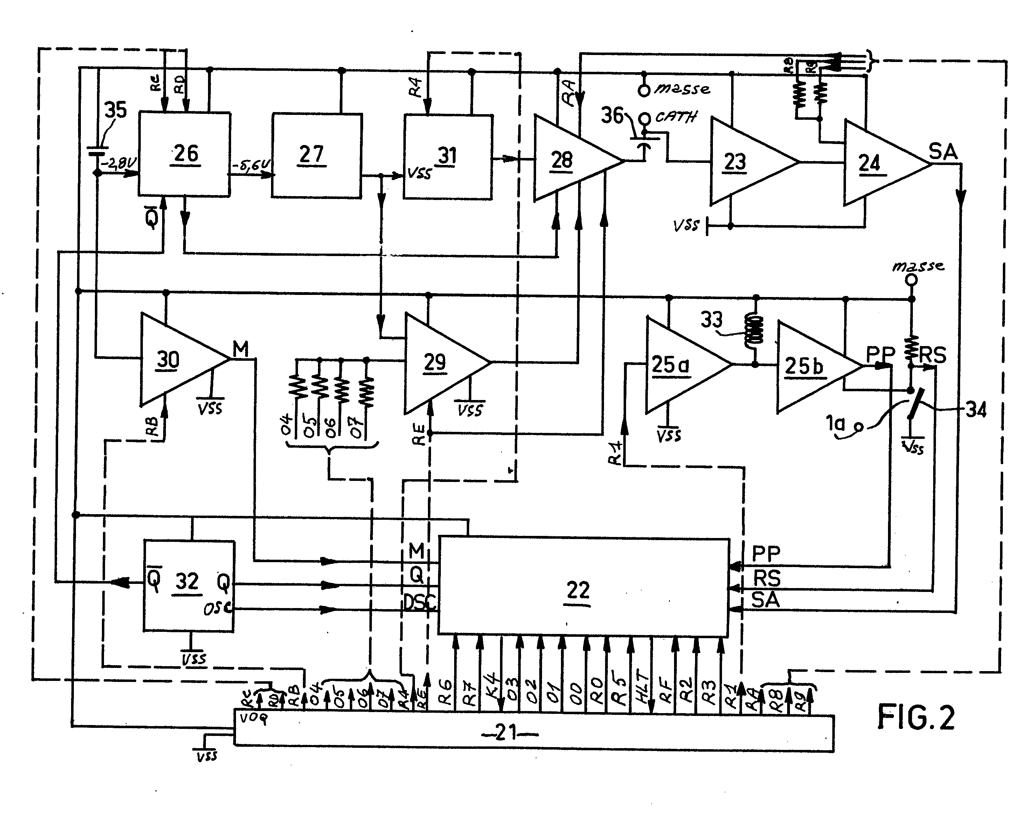

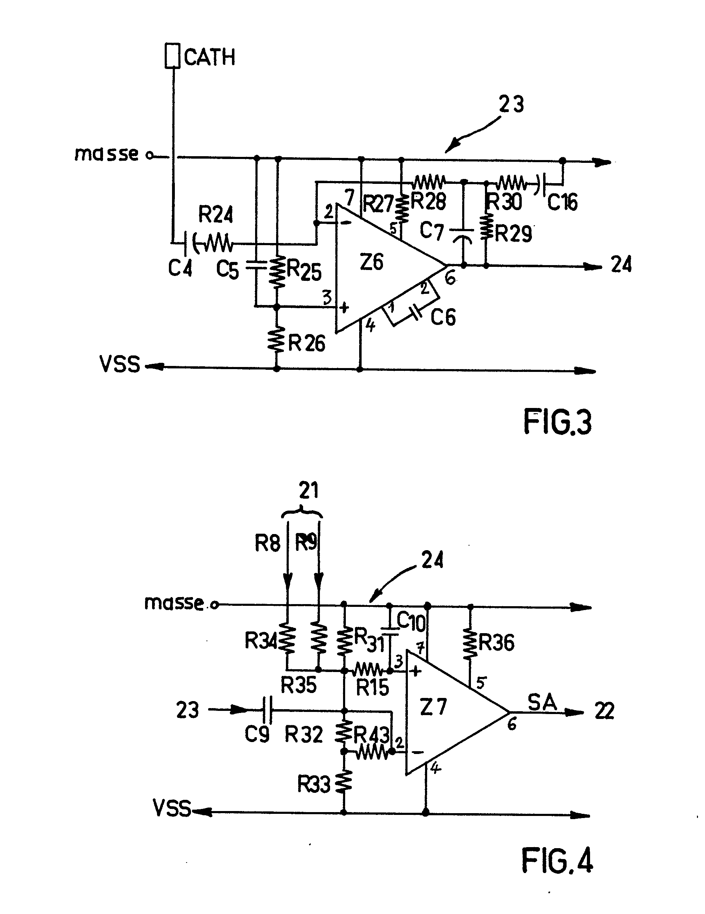

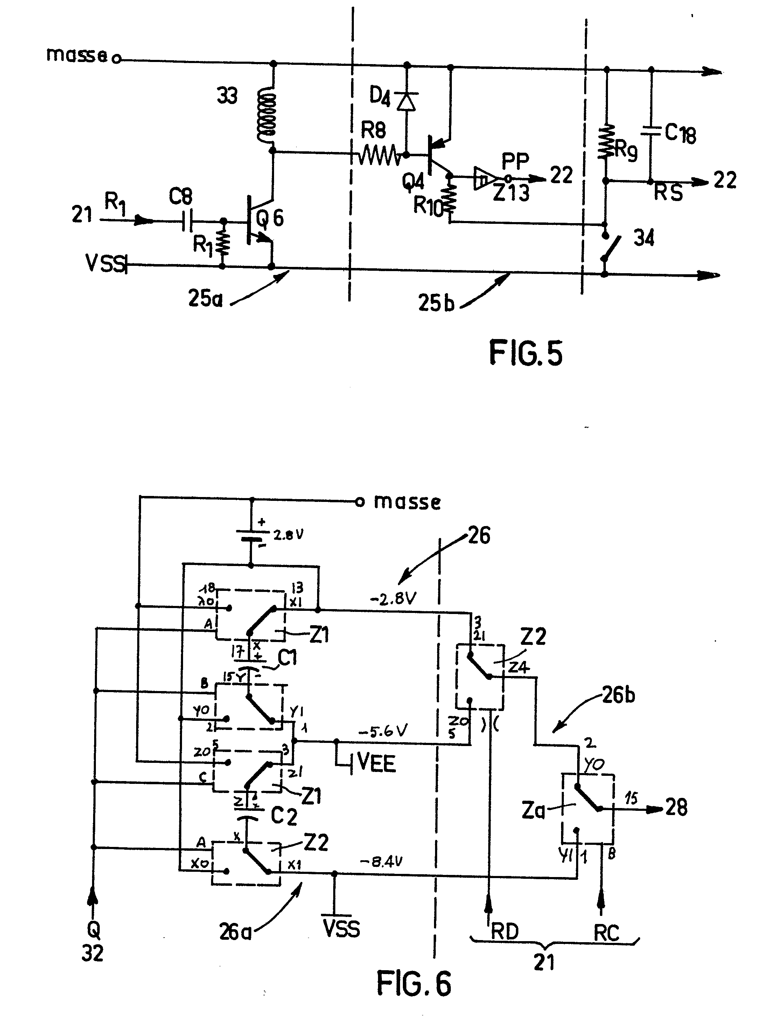

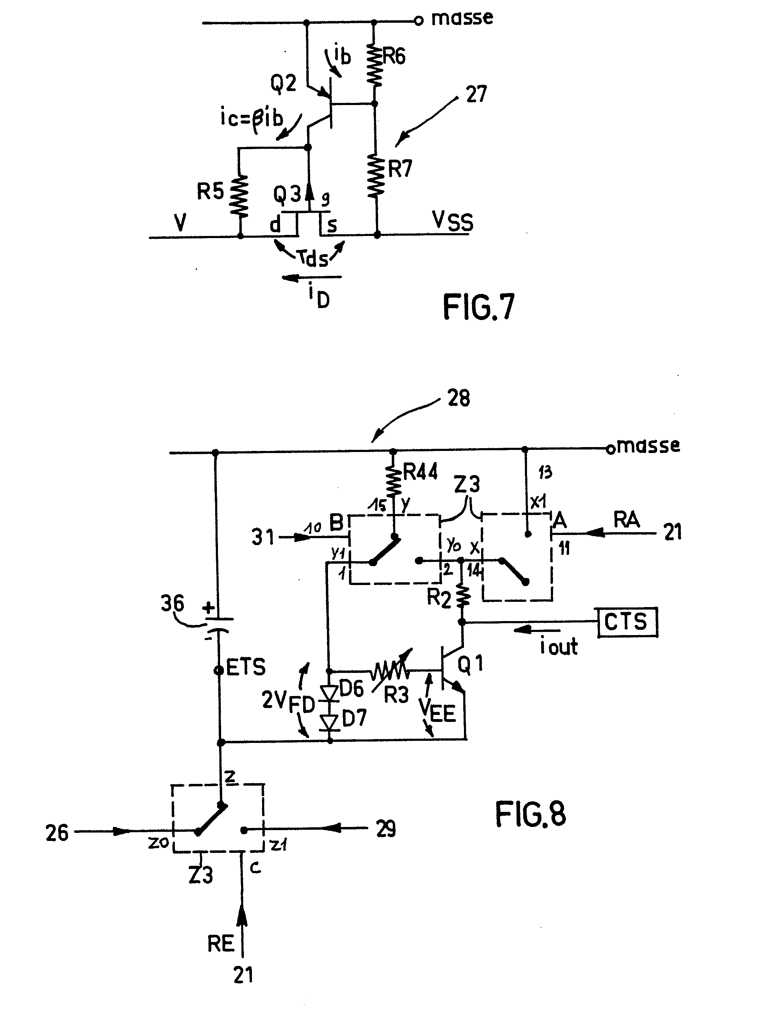

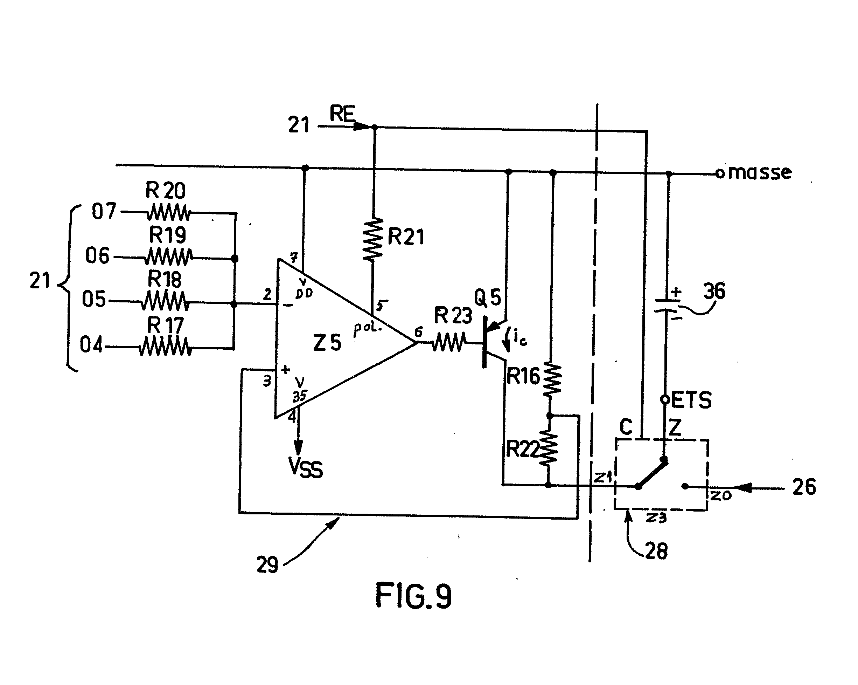

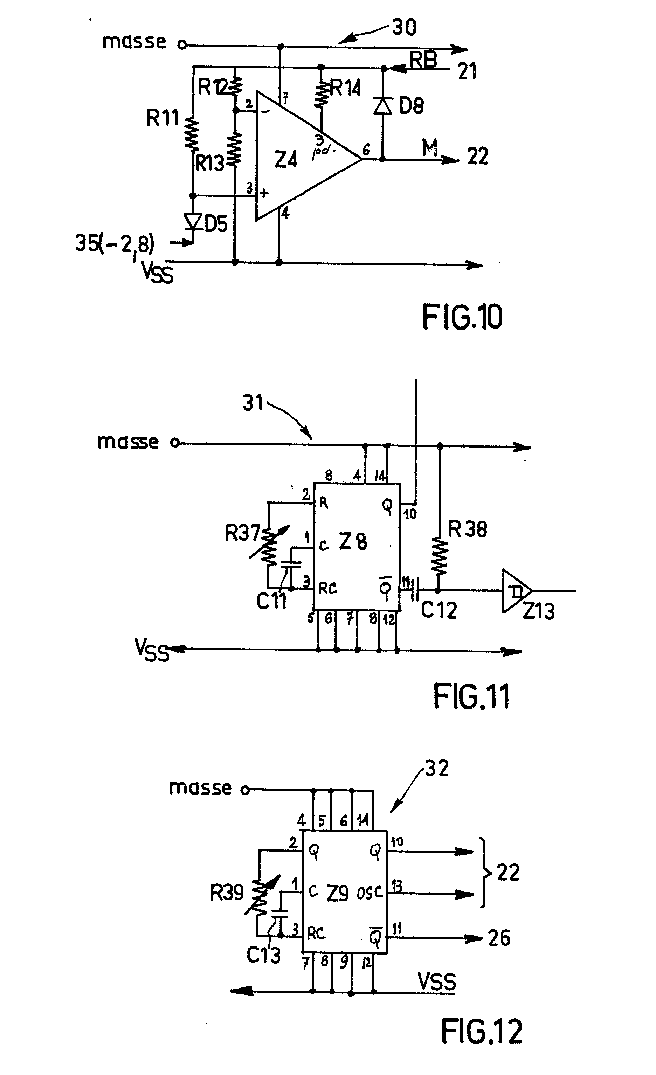

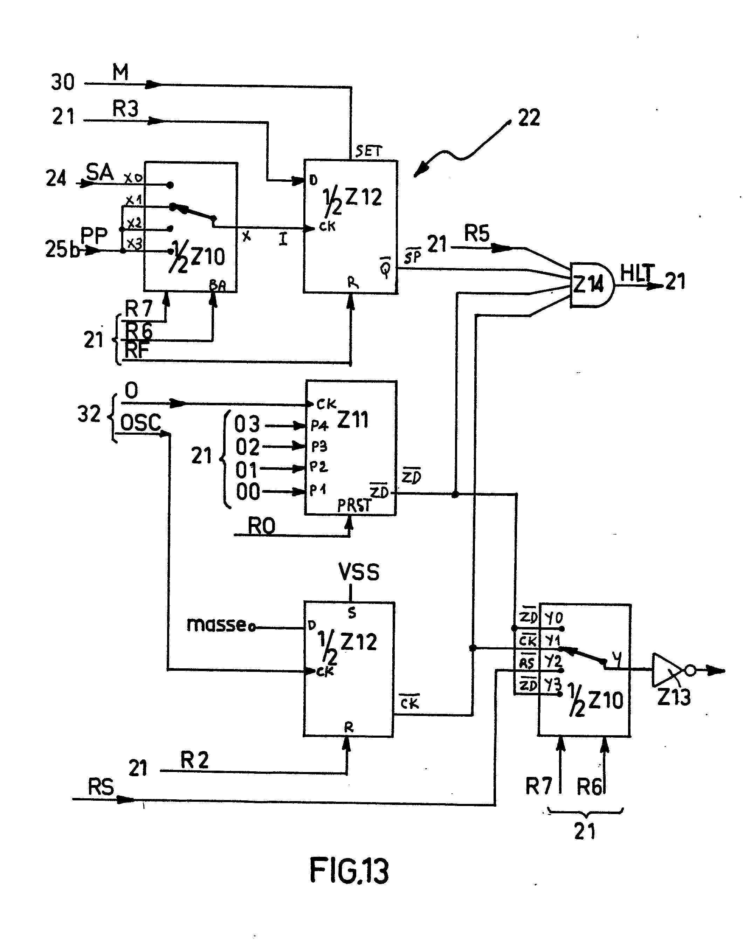

La figure 1 est une vue schématique du déroulement des modes opératoires du procédé ; la figure 2 est une vue schématique bloc d'un dispositif d'application du procédé selon la figure 1 ; la figure 3 est une vue schématique du circuit de l'amplificateur du dispositif de la figure 1 ; la figure 4 est une vue schématique du circuit du comparateur de détection du dispositif de la figure 1 ; la figure 5 est une vue schématique du circuit récepteur émetteur du dispositif de la figure 1 ; la figure 6 est une vue schématique du circuit tripleur de tension du dispositif de la figure 1 ; la figure 7 est une vue schématique du circuit régleur de tension du dispositif de la figure 1 ; la figure 8 est une vue schématique du circuit de sortie du dispositif de la figure 1 ; la figure 9 est une vue schématique du circuit d'essai du seuil du dispositif de la figure 1 ; la figure 10 est une vue schématique du circuit de comparateur de résistance de batterie du dispositif de la figure 1 ; la figure 11 est une vue schématique di circuit limiteur de rythme et de largeur du dispositif de la figure 1 ; la figure 12 est une vue schématique du circuit d'horloge du dispositif de la figure 1 ; la figure 13 est une vue schématique du circuit de l'interface de microprocesseur du dispositif de la figure 1.

Selon l'invention, il est proposé un procédé de commande et de réglage d'un appareil ou instrument alimenté électriquement, notamment médical et plus spécialement un stimulateur cardiaque implantable.

L'appareil comporte de manière connue une batterie d'accumulation d'énergie électrique d'alimentation, implantée et inaccessible à un contrôle direct de sa capacité d'alimentation résiduelle à un instant donné.

Ce stimulateur travaille en régime normal selon plusieurs modes opératoires normaux, avec des paramètres de fonctionnement, tel que tension de sortie, rythme, sensibilité et période réfractaire, préprogrammés.

Les divers modes de travail différent par leur réponse aux ondes envoyées par un programmeur extérieur au corps d'implantation du stimulateur, détectées par un amplificateur détecteur, entre le cathéter associé au stimulateur et la masse.

La commande de la mesure du degré de charge de la batterie, et des divers paramètres de fonctionnement de l'appareil, ainsi que le réglage de ces paramètres à des valeurs décidées par le médecin traitant du patient recevant le stimulateur, est obtenu en faisant passer le stimulateur sur des modes opératoires de commande, et ce en plaçant un aimant 1 extérieur au corps du patient au voisinage du stimulateur pour faire passer un commutateur de lecture d'un état ouvert à un état fermé temporaire durant jusqu'au retrait de l'aimant 1.

Le programmeur extérieur sert aussi à programmer les différents paramètres de fonctionnement du stimulateur.

Le stimulateur comporte trois modes opératoires normaux intitulés VOO, VVI et VVT (figure 1).

Le mode VOO est le mode de travail normal et permanent à rythme et tension de sortie constants, tels qu'antérieurement programmés. Le stimulateur est totalement insensible, indépendant de l'amplificateur détecteur et des impulsions programmées envoyées par télémétrie.

Dans le mode VVI la durée du cycle de fonctionnement du stimulateur comporte trois sous-périodes successives ; une première sous-période réfractaire absolue, immédiatement après le commencement d'un cycle de ce mode VVI, pendant laquelle le stimulateur ne répond pas aux signaux provenant de l'amplificateur détecteur ; une deuxième sous-période réfractaire
relative après la première sous-période, pendant laquelle
le stimulateur apprécie s'il convient ou non de passer
dans la sous-période suivante ; une troisième sous-
période d'alerte, dans laquelle le stimulateur répond à une
impulsion détectée par l'amplificateur de détection en commençant un nouveau cycle de fonctionnement mais sans générer
d'impulsion de sortie. En l'absence d'impulsions détectées
par l'amplificateur détecteur le stimulateur génère des impulsions de sortie au rythme programmé. Dans le mode VVI
un circuit à impédence d'entrée double est actif. La tension
de sortie, la sensibilité, le rythme et la durée de la période réfractaire absolue sont programmables. Le stimulateur
est insensible aux impulsions de programmation.

Le mode VVT n'est différent du mode VVI qu'en ce qui concerne
la réponse du stimulateur à une impulsion de sortie de l'amplificateur détecteur pendant la période d'alerte. En effet,
le stimulateur répond à une telle impulsion en arrêtant le
cycle de fonctionnement après un retard de synchronisation
préétabli puis en fournissant ensuite une impulsion de sortie.
Si le stimulateur demande une impulsion de sortie avant la
fin de la période de limitation du rythme qui suit l'impulsion
de sortie précédemment fournie, il est bloqué mais un
nouveau cycle commence malgré tout.

On fait passer le stimulateur à partir des modes VOO, VVI
ou VVT aux autres modes (flèches 2, 3, 4), en approchant
du stimulateur l'aimant 2 pour faire passer le commutateur
de lecture à partir de l'état ouvert à l'état fermé.

Le stimulateur comporte également six modes opératoires de
commande FMR, MAG, PRG, TEL, TSL, et VTL.

Lorsque l'aimant 1 est placé au voisinage du stimulateur,
fonctionnant en mode VOO, le stimulateur passe dans le premier mode opératoire FMR (flèche 3) à la fin du cycle en cours qui peut être défini par une impulsion de sortie ou bien une impulsion d'activité décelée pendant la sous-période d'alerte. Pendant le mode FMR, le stimulateur mesure la résistance interne de la batterie d'alimentation, qui est directement fonction de son état de charge. Si la résistance interne de la batterie, mesurée sous une charge connue est inférieure à une certaine valeur préétablie, l'impulsion de sortie suivante est délivrée à un certain rythme.

Si au contraire cette résistance est supérieure à cette valeur, l'impulsion de sortie suivante est délivrée à un rythme inférieur appelé rythme magnétique. L'impulsion de sortie à la fin du mode FMR, fait déjà partie du mode suivant MAG, et est délivré à la tension de sortie du programme précédent.

la durée du mode FMR est exactement identique à la durée dun cycle de rythme magnétique. Dans le mode FMR le stimulateur est insensible à l'amplificateur de détection au programmeur extérieur et au commutateur de lecture.

Le passage du mode FMR au mode MAG (flèche 5) intervient automatiquement immédiatement avant une impulsion de sortie établie lors du mode FMR, au rythme magnétique. Dans le mode MAG, le signal de sortie est à tension maximale et au rythme magnétique. Dans le mode MAG le stimulateur peut changer de mode selon les instructions reçues au programmeur (flèche A), dont les informations sont reçues par un amplificateur de programmation 2 associée à une antenne bobine, fonctionnant par couplage magnétique, comme un transformateur à noyau d'air. Si l'amplificateur de programmation reçoit une séquence de programmation valable selon le code de cette séquence, le stimulateur passe automatiquement dans l'un des modes de commande suivants (PRG, TEL, TSL, ou VTL). Si l'aimant est enlevé pendant la programmation, donc trop tôt, l'amplificateur de programmation émet immédiatement une impulsion qui annule la séquence en cours de progression. Une impulsion télémétrique est transmise au programmeur immédiatement après chaque impulsion du stimulateur fonctionnant en mode MAG et l'amplificateur de programmation est mis
hors service pendant un certain temps préétabli chaque fois
qu'une telle situation apparaît. Si lorsqu'il fonctionne
en mode MAG le code pour le passage en mode PRG est détecté
par l'amplificateur de programmation, le stimulateur passe
dans le mode PRG (flèche 6).

Dans le sixième mode PRG, le stimulateur reçoit du programmeur externe les informations de programmation (mode, période
réfractaire, rythme, sensibilité, l'amplitude des impulsions
de sorties). Ces informations sont codées pour éviter toute
fausse programmation. Si une erreur de programmation est
détectée, les mémoires du stimulateur ne sont pas modifiées
et le stimulateur revient au mode MAG (flèche 7). Si le programme reçu est correct, les données des mémoires du stimulateur sont modifiées pour correspondre aux valeurs programmées. A l'impulsion de sortie suivante le stimulateur passe
au mode TEL (flèche 8).

Dans le mode TEL, le stimulateur envoit des informations
au programmeur extérieur et lui fournit toutes les valeurs
programmées, le rythme magnétique et le rythme de base
(flèche B).

Le passage vers le mode TEL se fait soit, ainsi que déjà mentionné, à partir du mode PRG (flèche 8), soit en réponse à
la réception par le programmeur du code correct directement
à partir du mode MAG (flèche 9).

L'opération du mode TEL dure moins d'un cycle du rythme
magnétique et à la fin le stimulateur revient au mode MAG
(flèche 10).

Dans le mode TSL, on mesure le seuil de stimulation du patient
et à cet effet le stimulateur émet une série d'impulsions
(par exemple dix) de tension décroissante et au rythme magnétique. Le passage vers le mode TSL a lieu si le code
correspondant est reçu de la part du programmeur, lorsque le stimulateur se trouve encore dans le mode MAG (flèche 11).

Si l'aimant la est retiré pendant le fonctionnement en mode TSL (par exemple si le médecin veut arrêter le programme en mode TSL parce qu'il constate que le stimulateur ne reçoit plus), à l'impulsion suivante le stimulateur passe au mode VTL (flèche 12). Si l'aimant 1 est laissé en place, le stimulateur retourne, à la dixième impulsion du mode TSL, dans le mode MAG (flèche 13).

Dans le mode VTL, le stimulateur délivre un nombre préétabli d'impulsions au rythme programmé et d'amplitude maximale.

Le passage dans le mode VTL a lieu (à la première impulsion de sortie) après que l'aimant est retiré (la) pendant le fonctionnement du stimulateur en mode MAG ou TSL (flèches 11, 12) ou bien lorsque le code correspondant est reçu de la part du programmeur extérieur en mode MAG (flèche 14).

On sort du mode VTL si l'aimant 1 est en place après un nombre préfixé d'impulsions en mode VTL, par exemple à la quatrième impulsion. Le stimulateur retourne alors au mode MAG (flèche 15). Si l'aimant la n'est pas en place le stimulateur revient à celui des modes VOO, VVI ou VVT qui a été le dernier programmé (flèches 16, 17, 18).

On décrira à titre d'exemple non limitatif une forme de mise en oeuvre du procédé selon l'invention.

La durée de l'impulsion de sortie est fixée pour tous les modes à 0,5msec.

L'impulsion de sortie a une tension pouvant être programmée selon trois valeurs notamment basses ou faibles, normale et élevée correspondant respectivement à la tension de charge d'une capacité soit directement à partir de la batterie du stimulateur soit par un doubleur, soit par un tripleur de tension. Ces valeurs programmables sont par exemple approximativement 2,5 V, 5 V, 7,5 V. La valeur exacte dépend de la résistance interne de la batterie, de la résistance de charge du stimulateur et de son rythme. Le mode TSL comporte également comme indiqué une suite de dix valeurs de la tension. Dans les modes MAG, PRG, TEL et VRL la valeur élevée est automatiquement sélectionnée.

La limite de l'intensité des impulsions est par exemple à 20mA au niveau de tension élevée. Cette valeur est approximativement indépendante de la tension de l'impulsion. Le circuit de sortie est du type à décharge d'une capacité, et l'intensité des impulsions dépend de l'impédence de charge jusqu'à une limite préétablie.

Les rythmes d'impulsion peuvent être programmés à l'une d'un certain nombre (par exemple seize) de valeurs appelées rythmes programmés . Le rythme du stimulateur est celui programmé selon les modes VOO, VVI, VVT et VTL. Les valeurs de ces rythmes sont 30 impulsions par minute et de 50 à 120 impulsions par minute, de 5 en 5 impulsions par minute soit (50, 55, 60 ... 110, 115, 120).

Pour le rythme magnétique, il y a deux fréquences possibles, 86 impulsions par minute et 77 impulsions par minute.

Lorsque la résistance de la batterie telle que mesurée est inférieure à 20 KOhm, le rythme magnétique a la valeur élevée (86). Au contraire si cette résistance est supérieure à 20KOhm, le rythme magnétique est la valeur la plus basse (77), ce qui indique un épuisement imminent de la batterie.

Le stimulateur est en rythme magnétique dans les modes FMR, MAG, TEL, PRG et TSL. Le premier intervale d'impulsion dans le mode TSL est plus grand que le rythme magnétique réglé à une durée connue, importante, (par exemple 310 msec). De la sorte le début de la suite descendante d'impulsion de tension de mode TSL peut facilement être repéré sur un appareil externe.

Le stimulateur comporte une limite du rythme d'impulsions, à une valeur supérieure aux valeurs programmables (par exemple 135 impulsions par minute) quel que soit le mode de fonctionnement. Si par suite d'un défaut, il est demandé des impulsions de sortie à un rythme plus rapide, un limiteur de rythme permet de générer quelques impulsions de sortie, mais jamais une paire d'impulsions un rythme dépassant 135 impulsions par minute.

La détection n'est faite seulement que pour les modes VVI et VVT.

La sensibilité de l'appareil est programmable à plusieurs (par exemple quatre) valeurs notamment ; 1, 2, 3, et 4mV.

La période réfractaire absolue est programmable à plusieurs (notamment (deux) valeurs, notamment 165 msec et 320 msec. La période réfractaire absolue est une période réfractaire relative d'une durée fixe de 145 msec ce qui offre donc un choix de période réfractaire programmable de 310 ou 465 msec.

Dans les modes VVI et VVT, le stimulateur compte les impulsions de sortie de l'amplificateur de détection émis pendant la période réfractaire relative. Si le stimulateur détecte quatre telles impulsions, il devient insensible aux signaux émis par l'amplificateur de détection pendant le reste du cycle de fonctionnement. Si le stimulateur détecte moins de quatre impulsions (trois au moins), le stimulateur passe à la période d'alerte et complète le cycle de fonctionnement sous l'effet de l'amplificateur de détection. Le stimulateur fonctionne alors à rythme constant, en présence d'un bruit-répété au moins égale à 35Hz.

L'impédence à l'entrée du stimulateur est normalement de 16kOhm. Pendant les modes VVI et VVT, l'impédence à l'entrée est réduite, pendant une période de 20msec à 1,3 KOhm après le début de chaque cycle et ce pendant une durée de 100msec.

Cela permet de faire revenir l'amplificateur de détection à son état de repos après une impulsion de sortie en moins de 150 msec, quelle que soit la tension de sortie programmée et la sensibilité.

La durée entre une impulsion détectée par l'amplificateur de détection en période d'alerte et l'impulsion de sortie suivante du stimulateur correspond au rythme programmé, diminué d'un retard de synchronisation d'au plus 20,5 msec. Le stimulateur est dans le mode FMR pendant moins d'une seconde. Pendant les premières 164 msec, le stimulateur met la batterie sous une charge de 10KOhm, pendant lesquels tel qu'expliqué il est procédé à la vérification du degré de charge de la batterie et à la détermination du rythme magnétique.

Dans le mode MAG le rythme d'opération est le rythme magnétique et la tension est portée à la valeur élevée soit 7,5V.

L'amplificateur de programmation reçoit du programmateur extérieur des impulsions de 30 $\mu$sec, par une antenne-bobine en couplage magnétique comme indiqué.

Dans le mode TEL les informations envoyées au programmeur extérieur (flèche 3) le sont au moyen de la même antenne que celle utilisée à la réception, sous la forme d'une série d'impulsions toutes les 2 $\mu$sec, l'écart entre les impulsions comportant l'information. Cet écart donne également la fréquence de l' horloge pilote du stimulateur, pouvant être utilisée par le programmeur externe pour déterminer le rythme de stimulation exact et le rythme magnétique exact, aucun d'entre eux n'étant mesuré directement.

Le stimulateur envoyant au programmeur extérieur (flèche B) la période fondamentale de l'horloge pilote et également le contenu de la mémoire programmée, le programmeur peut fournir les valeurs du rythme programmé, du rythme magnétique et de la période réfractaire.

Lors de l'entrée dans le mode TSL, l'intervalle de temps jusqu'à la première impulsion de sortie est plus long de 310 msec que la durée du rythme magnétique. Toutes les autres impulsions suivantes sont au rythme magnétique. Il a déjà été indiqué que dans ce mode TSL, il y a dix impulsions successives du stimulateur. La première est à une tension de 5,5 V, la suivante à une tension de 5,0 V et ainsi de suite par pas de 0,5 V jusqu'à une tension de 1,0 V.

On décrira ensuite une forme d'exécution préférée mais non limitative d'un dispositif de commande et de réglage pour un tel appareil ou instrument alimenté électriquement, notamment un stimulateur cardiaque implantable.

Le dispositif comprend (figure 2) des circuits numériques soit un microprocesseur 21 et un circuit interface 22 reliant le microprocesseur 21 aux autres circuits du dispositif.

Les modes opératoires du stimulateur ne demandant que peu d'opérations logiques rapides par cycle de fonctionnement, le microprocesseur 21 est inactif, jusqu'à ce qu'une opération logique soit nécessaire.

Le dispositif comprend dix circuits analogiques 23 à 32 dont le rôle, le fonctionnement et les moyens qui les constituent sont décrits par la suite.

Le microprocesseur 21 comporte vingt-quatre sorties et deux entrées. Onze sorties, notamment RO, R2, R3, R5, R6, R7, RF, 00, 01, 02 et 03 commandent l'interface 22. Les treize autres sorties, notamment RC, RD, RB, 04, 05, 06, 07, R4, RE, RA, R1, R8 et R9 commandent le fonctionnement des circuits analogiques 23 à 32.

Six lignes de signaux M, Q, OSC, PP, RS et SA reviennent des circuits analogiques 23 à 32 vers les deux entrées du microprocesseur 21. L'une des deux entrées, soit HLT sert à maintenir le microprocesseur au repos. Le circuit interface 22 détermine laquelle des lignes de signaux doit être reliée à l'une des entrées K4 et HLT du microprocesseur 21. Le microprocesseur 21 et son circuit interface 22 constituent des moyens de commande au repos jusqu'à ce qu'un changement d'état approprié sur l'une des six lignes de séquence ne demande une décision logique. Le microprocesseur 21 répond à ce changement et peut modifier l'état de l'une ou de plusieurs de ses sorties que commandent les circuits analogiques 23 à 32. Lorsque l'opération logique est achevée, le microprocesseur 21 s'arrête et le circuit numérique attend qu'un autre signal vienne des circuits analogiques.

Les dix circuits analogiques sont les suivants ;

Un amplificateur de détection 23 qui amplifie les ondes R apparaissant entre le cathéter du stimulateur (borne CATH) et la masse et transmet le signal inverse et amplifiée à un comparateur de détection 24. L'amplificateur de détection 24 amplifie également les impulsions de sortie du stimulateur apparaissant à la borne CATH (notamment les impulsions négatives 8V, 0,5msec).

Un comparateur de détection 24 qui détecte si les impulsions en sortie de l'amplificateur de détection 23 sont positives ou négatives (notamment correspondent à des ondes R négatives ou positives au cathéter) pourvu que leur front de montée soit assez rapide. La sortie SA de ce comparateur est reliée à l'interface 22, entrée SA.

Un émetteur-récepteur 25a, 25b de communication avec le programmeur extérieur (non figuré) au corps du patient, à l'aide d'une antenne 33 située en dehors du circuit hybride. Il comporte une partie émetteur 25a et une partie récepteur 25b. La partie récepteur 25a est fonctionnelle uniquement si le commutateur de lecture 34 est fermé, notamment à l'aide de l'aimant 1 (non figuré) ; elle est commandée par

0064002

la borne RA du programmeur.

Un tripleur de tension 26 qui comprend une partie à fonction de tripleur de tension 26a et une partie à fonction de sélecteur de courant de sortie 26b.

La partie tripleur de tension reçoit le courant de sortie de la batterie 35 du stimulateur à -2,8V et fournit aux autres circuits un courant à -5,6V et -8,4V. La partie sélecteur du courant de sortie du circuit choisit entre les tensions possibles : -2,8V ; -5,6V ou -8,4V pour le courant de sortie du stimulateur, d'après les réglages des sorties RC et RD du microprocesseur 21.

Un régulateur de tension 27 qui reçoit le courant à -5,6V du tripleur de tension 26 et qui vournit en sortie à tous les circuits, un courant d'alimentation de 3,5 V sur une ligne d'alimentation au potentiel Vss (doubleur).

Un circuit de sortie 28 conformé, ainsi que décrit ultérieurement qui permet à un circuit d' essai de seuil 29 de décharger une capacité de stockage 36 de l'énergie de sortie à des niveaux de tension préétablis et éviter une recharge de cette capacité à partir du tripleur de tension 26 pendant le travail en mode TSL.

Le circuit d'essai du seuil 29 qui - en mode TSL - décharge la capacité de stockage 36 jusqu'à des niveaux de tension prédéterminés, selon l'état des sorties RE, 04, 05, 06 et 07 du microprocesseur 21.

Un comparateur de résistance qui mesure, en mode FMR la résistance interne de la batterie 30 ; il est commandé par le microprocesseur 21 notamment par sa sortie RB. Sa sortie M est reliée à l'entrée M de l'interface 22.

Un limiteur de rythme et de largeur d'impulsion 31 commandé par la sortie R4 du microprocesseur 21 dont la sortie est reliée à l'entrée du circuit de sortie 28.

Une horloge 32 qui est utilisée par l'interface 22 pour régler les sous-programmes de transmission et de réception. Elle comporte deux sorties Q et OSC vers l'interface 22 et une sortie $\bar{Q}$ vers le tripleur de tension 26.

On décrira ensuite en détail une forme de réalisation du microprocesseur 21, de l'interface 22 et des circuits analogiques 23 à 32.

L'amplificateur 23 (figure 3) présente un gain de crête de 60, sa bande passante est centrée sur 50 Hz et la largeur de bande est de 100 Hz. Il est constitué par un amplificateur Z6 de faible puissance dont le gain et la bande passante sont déterminés par un réseau de réaction constitué par des résistances R28, R29, R30 reliées électriquement par une extrémité en un point commun ; la résistance R28 étant reliée à la borne 2 de Z6 ; la résistance R29 reliée à la sortie 6 de Z6 ; et la résistance R30 reliée à une capacité C16 elle-même reliée à la masse. Une capacité C7 est montée en prallèle à R29. Le réseau d'entrée comporte une capacité C4 reliée à la borne CATH et à une résistance R24 elle-même reliée à la borne 2 de l'amplificateur. L'entrée sans inversion de Z6 est maintenue à une tension d'approximativement 0,5 V par des résistances R25 et R26 en série reliant Vss et la masse. La capacité C5 est montée en parallèle avec R25. La borne 3 de Z6 est reliée aux sorties adjacentes de R25 et R26. Une résistance R27 est reliée à la borne 5 de Z6 et à la masse et sert à ramener le courant de repos à la valeur de repos soit 1 $\mu$. Une capacité C6 reliée en parallèle entre les bornes 1 et 2 de Z6 sert à la compensation. Les valeurs des composants sont choisies de sorte que la bande passante de l'amplificateur Z6 soit déterminée uniquement par les réseaux rétroactifs et l'entrée et non pas par le gain. La résistance R24 limite le courant d'entrée à 40 $\mu$A. La constante de temps de 22 msec de la résistance R24 et la capacité C4 protège la capacité C4 d'un renversement de polarité pendant les impulsions de 0,5msec. La sortie de l'amplificateur récupère jusqu'à 30mV de sa valeur de repos en 150msec après une impulsion de sortie du stimulateur.

Le comparateur de détection 24 (figure 4) consiste en un amplificateur Z7 de faible puissance commandé sans compensation ni rétroaction avec un courant de repos de 1 $\mu$A déterminé par une résistance R36 reliée entre sa borne 5 et la masse. Un réseau de polarisation d'entrée est constitué par des résistances R31, R32, R34 et R35 reliées en un point commun de la résistance R33 d'une part en série avec R32 et d'autre part branché sur Vss fixe la tension de seuil du comparateur, qui peut être commandée par les sorties R8, R9 du microprocesseur 21. Le réseau de couplage à l'entrée constitué par une capacité C9 reliée à la borne 2 de Z7 et la résistance R43 entre cette borne et le point commun des résistances R32 et R33 associe la sortie de l'amplificateur palpeur 23 à l'entrée d'inversion du comparateur 24. La capacité C10 entre la borne 3 de Z7 et la masse et la résistance R15 entre le point commun des résistances R31, R32, R34, R35 et la borne 3 de Z7 constituent une dérivation de l'entrée sans inversion à la masse. Le signal de sortie du comparateur est normalement élevé et est envoyé à un déclencheur Schmitt Z13 d'inversion et de mise en forme des impulsions. En sortie du déclencheur Schmitt il y a un signal présentant un front de montée lorsque sur la borne 2 de l'amplificateur Z7 est appliquée une impulsion dépassant le seuil de tension préétabli. Le réseau C9, C10, R43, R32 et R15 différencie le signal de l'amplificateur de détection avant que de l'appliquer à la borne 2 de l'amplificateur Z7 avec une constante de temps de 60msec, ce qui permet au comparateur de détecter les impulsions positives ou négatives émises par l'amplificateur de détection à condition que leur front de montée soit suffisamment rapide. Le microprocesseur 21 règle la sensibilité du stimulateur aux ondes R en réglant le seuil du comparateur de détection en reliant les résistances R34 et R35 soit à la masse soit au potentiel Vss, par ses sorties R8, R9, suivant le tableau suivant :

| R8 R9 du microprocesseur | | Seuil de Z7 | Sensibilité du stimulateur. |
|---|---|---|---|
| Vss | Vss | 30mV | 1mV |
| Vss | masse | 60mV | 2mV |
| masse | Vss | 90mV | 3mV |
| masse | masse | 120mV | 4mV |

La sensibilité du stimulateur est donnée pour une impulsion triangulaire négative de 10msec ou pour une impulsion triangulaire positive de 20msec sur le cathéter. Le gain de l'amplificateur de détection 23 pour ces impulsions est d'approximativement 30.

L'émetteur récepteur 25a, 25b (figure 5) comprend une partie émetteur 25a et une partie récepteur 25b. La partie 25a comprend un transistor Q6 dont l'émetteur est relié à Vss, le collecteur relié à l'antenne 33 elle-même reliée à la masse, et la base reliée d'une part à une résistance R1, elle-même mise au potentiel Vss et d'autre part à une capacité C8, reliée à la sortie R1 du microprocesseur 21, par laquelle cette partie émetteur 25a est commandée. La sortie R1 est normalement faible, le transistor Q6 est bloqué et le circuit ne consomme pas d'énergie. Sous l'effet d'une impulsion positive de la sortie R1, Q6 devient passant et charge la capacité C8 en environ 2 µsec puis se bloque de nouveau. Une impulsion de -3V est appliquée sur l'antenne 33 par le transistor passant Q6 et une impulsion d'environ +10V se produit lorsque le transistor Q6 se bloque. La résistance R1 décharge la capacité C8 lorsque le signal en sortie R1 diminue. Le circuit consomme 1 µA en transmettant dix impulsions à la seconde.

La partie récepteur 25b comprend un transistor Q4 dont la base est reliée d'une part à la masse à travers une diode
D4 et d'autre part à une résistance R8 elle-même reliée
à l'antenne 33 de la partie émetteur 25a ; dont l'émetteur
est reliée à la masse ; dont le collecteur est reliée d'une
part à une résistance R10 et d'autre part à un déclencheur
Schmitt Z13. La résistance R10 est reliée, par ailleurs et à
l'opposé au commutateur de lecture 34, lui-même relié du
côté opposé à Vss. Le commutateur 34 est normalement ouvert
lorsqu'il n'y a pas d'aimant 1 près du stimulateur. La résistance de charge R10 est reliée à la masse par une résistance
R9. Même si le tranristor Q4 est passant, le signal en sortie
du déclencheur Z13 est faible le circuit ne consume pas de
courant. Lorsque l'aimant 1 provoque la fermeture du commutateur 34, la résistance R10 est reliée à la masse, le signal
en sortie du déclencheur Z13 devient élevé, le transistor
Q4 devient passant et envoit une impulsion négative à l'interface 22 à travers le déclencheur Z13. Etant donné que l'interface 22 se déclenche sous l'effet des fronts de montée provenant du déclencheur Z13, on détecte le blocage du transistor Q4. Le transistor Q4 est rendu passant par une impulsion
négative sur l'antenne 33 dépassant la différence de potentiel base émetteur du transistor Q4  soit environ notamment
-0,5V. La diode D4 protège le transistor Q4 contre les impulsions positives sur l'antenne 33 et la résistance R8 protège
le transistor Q4 et la diode D4 contre les impulsions d'amplitudes élevées. A chaque fois que le microprocesseur 21
règle sa sortie R1 pour émettre une impulsion celle-ci est
détectée par la partie récepteur 25b. La durée de récupération de la partie récepteur 25b après une impulsion détectée est limitée par les capacités parasites base-collecteur
et émetteur collecteur du transistor Q4 ainsi que par la
capacité d'entrée du déclencheur Z13 qui doit se charger
par la résistance R10. Ces capacités sont en tout d'environ
30 pf, la durée de récupération étant de l'ordre de 30 μsec.

La ligne d'impulsion R5 à haute impédence vers l'interface
22 et à partir de la résistance R0 est bipassée par une capacité C18 vers la masse, pour éviter les problèmes d'accouplement parasites sur cette ligne.

Le tripleur de tension 26 et sélecteur de courant de sortie (figure 6) comprend les deux commutateurs Z1 et Z2, et les deux capacités C1 et C2 montées entre les bornes d'entrée de la languette mobile de ces commutateurs. La partie tripleur 26a est commandée par le signal Q provenant de l'horloge 32 qui a un cycle de 20ms et un coefficient d'utilisation de 50 % . Lorsque le signal Q est faible, les condensateurs C1, C2 et la batterie 35 sont montés en série et reliés au circuit extérieur. Sur la figure 6, la partie gauche représente le tripleur de tension 26a et la partie de droite la partie sélecteur du courant de sortie 26b. Chacun des commutateurs Z1 et Z est en positions X1, Y1, Z1 lorsque le signal de commande respectif A, B, C a la valeur logique 1 et en positions X0, Y0, Z0 lorsque le signal de commande A, B, C a la valeur logique 0. Le niveau de la valeur logique 1 est la masse. Le niveau de la valeur logique 0 est Vss = -3,5V. Le signal $\overline{Q}$ est une onde rectangulaire de 20ms de période, qui commande le tripleur 26 pour décharger sur la valeur logique 1 (tel que figuré sur le dessin). Les sorties RC et RD du microprocesseur 21 sélectionnent la tension de sortie selon le tableau suivant (on a considéré le cas -2,8V) :

| RC | RD | Tension de sortie (V) |
|----|----|----|
| 0 | 0 | -5,6 |
| 0 | 1 | -2,8 |
| 1 | 0 | -8,4 |
| 1 | 1 | -8,4 |

Le régulateur de tension 27 (figure 7) comprend un transistor Q2 dont l'émetteur est relié à la masse, la base reliée par une résistance R6 également à la masse et par une résistance R7 au potentiel Vss, et le collecteur étant par une résistance R5 à un potentiel V et également relié à un transistor à double base Q3 notamment à son émetteur, l'une des bases étant mise au potentiel Vss et l'autre base mise au potentiel V. Le courant d'alimentation Vss est de 5 $\mu$A. Vss est ajusté à la fabrication du circuit par un réglage dynamique de la résistance R6. Vers la fin de vie du stimulateur, la tension Vss est inférieure ou égale à -3V, lorsque la batterie de -2,8V est à une tension tombée à -1,8V.

Le circuit de sortie 28 comprend (figure 8) trois commutateurs analogiques X, Y, Z faisant partie du commutateur Z.3.

Le commutateur X est relié par sa borne $X_1$ à la masse par sa borne A à la borne RA du microprocesseur 21 et par sa borne X à la borne Y0 du commutateur Y, dont la borne Y est reliée à travers une résistance R44 à la masse, la borne B reliée au circuit de largeur d'impulsions 31 (figure 2), la borne Y1 reliée d'une part à travers deux diodes D6 et D7 en série à la borne Z du commutateur Z et d'autre part en parallèle à travers une résistance R3 à la base d'un transistor Q1 dont le collecteur est relié d'une part à CTS et d'autre part à travers une résistance R2 aux bornes X et Y0 des commutateurs X et Y, l'émetteur du transistor Q1 étant relié également à la borne Z du commutateur Z elle-même reliée à la borne ETS vers la capacité 36, la borne Z0 étant reliée au tripleur de tension 26, la borne Z1, reliée au circuit d'essai de seuil 29 de la borne C à la borne RE du microprocesseur 21.

Le commutateur Z est normalement en position Z0 ; la capacité externe 36 de 15 $\mu$F de stockage de l'énergie de sortie es chargé sous la tension choisie par le circuit de sélection de tension de sortie 29. Pendant le programme en mode TSL et d'essai du seuil, la sortie RE du microprocesseur 21 est réglée à la valeur 1 et la commutateur Z passe à la position Z1 (figure 9), ce qui permet au circuit d'essai de seuil de décharger la capacité de stockage 36 de l'énergie de sortie au niveau de tension préétablie et évite la recharge du condensateur à partir du tripleur 26.

Le commutateur Y est normalement en position Y0 et le signal à la sortie du circuit de largeur de l'impulsion 31 est faible. Dans cette position, les résistances R44 et R2 sont montées en série entre CTS et la masse, rendant l'impédence d'entrée du stimulateur au moins égale à 16KOhm. Lorsque le circuit de largeur d'impulsion 31 demande une impulsion de sortie du stimulateur en mettant sur la valeur 1 l'entrée B de commande du commutateur Y, celui-ci passe à la position Y1. Le transistor Q1 est rendu passant par l'impulsion du courant à travers la résistance R3 et il émet une impulsion de sortie du stimulateur. Lorsque le signal d'entrée B revient à la valeur 0, le commutateur Y revient à la position Y0 et les diodes D6 et D7 bloquent le transistor Q1. Ces diodes règlent la tension du point de jonction de la résistance $R_3$ et de la diode D6, rendant la limite du courant de sortie approximativement indépendante de la tension de sortie du stimulateur. Cela est nécessaire dans le programme d'essai de seuil TSL, où la tension de sortie ne dépasse pas 1 volt. Le fait que le commutateur Y déconnecte la résistance R44 de la sortie pendant l'impulsion de sortie réduit la consommation du circuit de manière importante, aux tensions de sorties élevées.

Le commutateur X est normalement ouvert (position X). Lorsque le microprocesseur 21 règle la sortie RA, le commutateur X se ferme et connecte la résistance R2 entre CTS et la masse, l'impédence d'entrée du stimulateur étant au moins égale à 1000 Ohms. La sortie RA est réglée sur 20msec au delà du front de montée de l'impulsion de sortie et est remise à la valeur 0, 100 msec plus tard, et ce uniquement pendant les modes VVI et VVT. Ainsi, la capacité d'accouplement de sortie est déchargée, ce qui procure une sensibilité élevée au stimulateur, en combinaison avec une tension de sortie élevée et une durée de récupération de 150msec. La résistance est ajustée dynamiquement pour une limite d'intensité de sortie de 20mA à 8V.

Le circuit de mesure du seuil 29 (figure 9) comprend schématiquement un comparateur Z5 dont l'entrée 2 est reliée par des résistances R17, R18, R19 ou R20 en parallèle à chacune des sorties 04, 05, 06, 07 du microprocesseur 21. L'entrée 5 du comparateur Z5 est reliée par une résistance R21 à la sortie RE du microprocesseur 21. La sortie 6 de Z5 est reliée par une résistance R23 à la base d'un transistor Q5 dont l'émetteur est relié à la masse et le collecteur à travers deux résistances R22 et R16 également relié à la masse. L'entrée 3, +, du comparateur Z5 est reliée au point commun des deux résistances R22, R16. Le collecteur du transistor Q5 est relié au circuit de sortie 28, notamment à la borne Z1 du commutateur Z (voir figure 8). La capacité extérieure de stockage 36 est chargée à une tension d'environ 8V, avant le début du programme. Les sorties RE, 04, 05, 06, et 07 du microprocesseur 21 sont à la tension Vss. Le comparateur Z5 n'est pas polarisé par l'intermédiaire de la résistance R21 et le commutateur Z est en position Z0. Le circuit de consomme alors pas d'énergie. Immédiatement après la première impulsion de sortie du programme en mode TSL, la sortie RE devient d'une tension élevée, rendant le comparateur Z5 passant. Les sorties 04, 05, 06, 07 sont réglées par le microprocesseur 21 de sorte que Z5 décharge la capacité 36 jusqu'à ce que la tension soit de 5,5 V. Après l'impulsion de sortie suivante, les sorties 04,05, 06,07, sont réglées pour décharger la capacité 36 jusqu'à ce que la tension soit de 5V. Cela continue par intervalles de 0,5V pendant 10 impulsions dont la plus faible est à 1V, après quoi la sortie RE et les sorties 04, 05, 06 et 07 retournent à la tension Vss. Immédiatement après une impulsion du programme d'essai du seuil TSL, la sortie 6 du comparateur passe à la tension Vss, ce qui charge la capacité 36 à travers le collecteur du transistor Q5, jusqu'à ce que la tension à la borne 3 du comparateur Z5 soit plus positive que la tension de référence réglée par les sorties 04, 05, 06, 07 à la borne 2 du comparateur Z5. Cette tension est proportionnelle à la tension de sortie du stimulateur. La tension de sortie du comparateur Z5 retourne alors à la masse jusqu'à ce que

0064002

les sorties 04, 05, 06, 07 soient modifiées après l'impulsion de sortie suivante. La résistance R23 empêche la capacité 36 de se décharger trop rapidement.

Le comparateur de la résistance de la batterie 30 (figure 10) comprend un comparateur Z4 de faible puissance, relié à l'entrée 2, - , par une résistance R13 du potentiel Vss et relié par une résistance R 12 . d'une part à la borne RB du microprocesseur 21 et d'autre part en premier lieu par une résistance R11 à l'entrée 3+ du comparateur Z4 et en second lieu à travers une diode D5 à la sortie -2,8 V de la batterie 35. Le circuit comporte ensuite une résistance R14 reliée à la borne 5 de polarisation du comparateur Z4 et à la sortie RB du microprocesseur 21 ; et une diode D8 reliée d'une part à la sortie RB du microprocesseur 21 et d'autre part à l'entrée M de l'interface 22. Lorsque le stimulateur n'est pas un mode FMR, la sortie RB du microprocesseur 21 est à la tension Vss ; le comparateur Z4 n'est pas polarisé le circuit ne consomme pas d'énergie. La diode D8 bloque la sortie 6 du comparateur Z4 à faible tension pour éviter un accouplement parasite avec ce point de haute impédence. La diode D5 est polarisée en sens inverse et n'est pas conductrice. Lorsque le stimulateur passe en mode FMR, le circuit met le circuit interface 22 à un potentiel élevé à la sortie du comparateur Z4, si la résistance interne de la batterie 30 est supérieure à 170 Ohms, ce qui signifie comme déjà décrit, que le stimulateur est proche de la fin de sa période de travail possible (fin de vie). La mesure de résistance interne de la batterie est réalisée en prélevant une intensité de courant relativement importante depuis la batterie 35, à travers la diode D5 et la résistance R11 lorsque la sortie RB du microprocesseur 21 est à une tension élevée. La tension à la borne 3 du comparateur Z4 devient de plus en plus positive au fur et à mesure que la résistance interne de la batterie augmente. Lorsque cette tension est plus positive que la tension de référence à la borne 2 un comparateur Z4 assurée par les résistances R12 et R13, le comparateur Z4 signale l'apparition des conditions d'épuisement proche par un signal de sortie élevé. Lors du travail en mode FMR, le microprocesseur 21 règle la sortie RB sur 160 msec et le circuit d'interface 22 du microprocesseur choisit le signal de sortie du comparateur Z4 après 140msec. Ainsi, le microprocesseur 21 ne choisit pas l'impulsion qui se forme à la sortie du comparateur Z4 immédiatement après avoir été polarisé.

Le limiteur de rythme et de largeur d'impulsion 31 (figure 11) comporte un multivibrateur monostable non redéclenchable 28, dont les entrées 2 et 3 sont reliées par une résistance variable et réglable R37 ; les entrées 1 et 3 sont reliées par une capacité $C_{11}$ ; la sortie $\overline{Q}$ reliée à travers une capacité $C_{12}$ , à un déclencheur Schmitt Z13 ainsi qu'à la masse à travers une résistance variable et réglable R38. Le rythme d'impulsion est déterminée par la résistance R37 et la capacité C11. La largeur d'impulsion est déterminée par la résistance et la capacité $C_{12}$ .

Le déclencheur Schmitt Z13 est branché de manière à fournir une impulsion positive lorsque Z8 est déclenché.

Le microprocesseur 21 demande une impulsion de sortie du stimulateur réglant la résistance de sortie R4 qui déclenche Z8 puis remettant R4 à zéro; Le signal de sortie $\overline{Q}$ diminue et la sortie Z13 augmente jusqu'à ce que le condensateur $C_{12}$ soit chargé à travers la résistance R38 ce qui fait redescendre le signal Z13. Si le microprocesseur 21 règle R4 de nouveau de manière erronée avant que la période de réglage préalable de Z8 ne soit écoulée, rien ne se passe. C'est ainsi que le rythme d'impulsion de sortie est limité à une valeur plus grande que celle de Z8. La résistance R37 est réglée dynamiquement pour qu'elle limite le rythme à 135 périodes par minute au début de la vie du stimulateur. La résistance R38 est réglée dynamiquement pour donner une largeur d'impulsion de $0,5 \pm 0,05$msec.

L'horloge 32 (figure 12) comprend un multivibrateur astable Z9, dont la période est déterminée par une résistance variable R39 reliée aux bornes 2 et 3 et une capacité C13 reliée aux bornes 1 et 3. La sortie Q (borne 10) fait avancer le compteur Z11 de l'interface 22 ; la sortie $\overline{Q}$ (borne 11) décharge le tripleur 26 ; la sortie OSC (borne 13) a une fréquence double de celle des sorties Q et $\overline{Q}$ et est utilisée par le circuit d'interface 22 pour régler les sous-programmes de transmission et réception.

Le circuit interface 22 (figure 13) comprend un multiplexeur Z10, un compteur Z11, un double flip-flop Z12 et une porte à quatre entrées Z14 "ET".

La borne X de la moitié du multiplexeur Z10 est reliée par la ligne SA au comparateur de détection 24 ; les trois bornes X1, X2, X3 sont reliées par la ligne PP à la sortie de l'émetteur 25b ; les deux autres bornes ou commande sont reliées aux bornes R6 et R7 au microprocesseur 21 ; la borne X est reliée à la borne CK de la moitié du flip-flop Z12 dont la borne D est reliée à la sortie R3 du microprocesseur 21 ; la borne 6 reliée par la ligne M à la sortie du comparateur de résistance de batterie 20 ; la borne R à la sortie RF du microprocesseur 21 ; la borne $\overline{Q}$ reliée à l'une des entrées de la porte Z14. L'autre demi flip-flop Z12 est relié par la borne S au potentiel Vss, par sa borne D à la masse ; par sa borne CK à la sortie OSC de l'horloge 32 ; par sa borne R à la borne R2 au microprocesseur 21 ; par la borne CK à une dernière entrée de la porte Z14 et aussi à la borne Y1 de l'autre demi multiplexeur Z10 -, relié également aux sorties R6 et R7 du microprocesseur 21 et par sa borne Y au déclencheur Z13. Le compteur Z11 est relié par sa borne CK à la sortie Q à l'horloge 32 ; par sa borne PRST à la sortie RO du microprocesseur 21. Par sa borne $\overline{ZD}$ d'une part aux bornes Y0 et Y3 et d'autre part à une troisième entrée de la porte Z14 dont la dernière entrée est reliée à la sortie R5 au microprocesseur 21 et dont la sortie est reliée à la sortie HLT du microprocesseur 21. Le circuit interface 22 réalise deux fonctions ; programmation et commande d'arrêt.

Le programmeur comprend le compteur de descente Z11 préréglable, à quatre bits, qui effectue un compte à rebours pour chaque front de montée de l'horloge 32. Quant un comptage nul est atteint, la borne $\overline{ZD}$ de Z11 prend une valeur faible.

Le dispositif de commande vise à régler l'entrée HLT du microprocesseur 21 à une valeur élevée lorsqu'il n'y a pas d'opération logique à réaliser et à le faire redémarrer lorsqu'une telle opération est demandée. Le microprocesseur 21 est déclenché lorsque l'une des quatre entrées de Z14 prend une valeur faible ce qui se produit lorsque la borne $\overline{ZD}$ de sortie du compteur Z11 ou la sortie $\overline{Q}$ de l'un des flip-flop de Z12 prend une valeur faible.

La borne $\overline{ZD}$ du compteur Z11 prend une valeur faible lorsque le comptage d'un - nombre pré-établi de cycles de l'horloge 32 est terminé.

La borne $\overline{CK}$ du flip-flop Z12 devient faible sur un front de montée de la sortie OSC de l'horloge 32, lorsque la borne R2 est faible. $\overline{CK}$ devient élevé de nouveau lorsque le microprocesseur 21 remet à zéro de nouveau le flip-flop Z12 en mettant une impulsion sur la sortie R2.

La borne $\overline{SP}$ du flip-flop Z12 prend une valeur faible sur un front de montée de l'entrée $\overline{CK}$ (ou de la sortie X du multiplexeur 20), à condition que les sorties R3 et RF du microprocesseur 21 soient respectivement élevées et faibles. La borne $\overline{CK}$ est connectée soit à la sortie SA du comparateur de détection 24 soit à la sortie PP de l'amplificateur pour programme 25b selon les états de sortie R6 et R7 du microprocesseur 21. La borne $\overline{SP}$ devient également faible sous l'effet d'un signal élevé fourni par la sortie M du comparateur Z4 de la résistance de la batterie 30 (figure 10) connecté à l'entrée SET du flip-flop Z12. $\overline{SP}$ devient de nouveau élevé lorsque le microprocesseur 21 remet le fli-flop Z12 à zéro en réglant une impulsion de sortie RF.

Ainsi, la commande d'arrêt, peut faire démarrer le microprocesseur 21 en réponse à un nombre préétabli de cycles de la sortie Q de l'horloge 32, ou en réponse à un seul cycle de la sortie OSC de l'horloge 32, en réponse à des signaux émis par l'amplificateur de détection (sortie) par l'amplificateur pour programme (sortie PP) et par le comparateur de résistance de la batterie (sortie M).

Le mode de fonctionnement du multiplexeur est le suivant :

| Valeur de la sortie R7 du micro-processeur 21 | Valeur de la sortie R6 du micro-processeur 21 | Liaison de X avec | Liaison de Y avec |
|---|---|---|---|
| 0 | 0 | SA | ZD |
| 0 | 1 | PP | CK |
| 1 | 0 | PP | RS |
| 1 | 1 | PP | ZD |

Les six lignes de signalisation arrivant de l'interface 22 à partir des circuits analogiques sont donc les suivants : (figure 2)

1 - La sortie Q de l'horloge 32 (borne 10 de Z9)

2 - la sortie OSC de l'horloge 32 (borne 13 de Z9)

3 - La sortie SA du comparateur de détection 24 (borne 6 de Z7).

4 - La sortie RS associée au commutateur de lecture 34

5 - La sortie PP du récepteur 25b (collecteur du transistor Q4).

6 - La sortie M du comparateur de la résistance de la batterie 30 (borne 6 de Z4).

Le microprocesseur 21 est d'un type approprié connu ou non en soi. Il retient indéfiniment, à l'état d'arrêt, le contenu des registres visibles par l'utilisateur.

Une fois démarré, le microprocesseur 21 doit définir lequel des signaux du circuit analogique est nécessaire pour l'opération logique et qui est obtenu à l'aide de l'entrée K4 que le microprocesseur 21 soumet à essai pendant son fonctionnement. Le multiplexeur d'entrée K4 (1/2 Z10) peut connecter à K4 soit la sortie ZD du rythmeur, soit la sortie CK du flip-flop CK, selon les états des sorties R6 et R7. Si en essayant K4, le microprocesseur 21 trouve qu'aucun de ces signaux ne l'a déclenché, il considère par élimination que cela résulte du signal $\overline{SP}$ du flip-flop Z12. Le microprocesseur peut déterminer lequel des signaux SA, PP ou M a réglé le flip-flop Z12 en considérant les états de sorties R3, R6, R7 et RB. Le microprocesseur 21 peut également utiliser K4 pour savoir si le commutateur de lecture 34 est ouvert ou fermé.

La première opération effectuée par le microprocesseur 21 lorsqu'il démarre est de remettre la sortie R5 à zéro. Le microprocesseur 21 détermine alors quel est le signal du circuit analogique qui l'a déclenché et exécute une opération logique conformément aux instructions fournies par sa mémoire morte. Les sorties R0, R2 et RF sont utilisées pour remettre à zéro le programmeur ou le flip-flop qui l'a démarré. Lorsque l'opération logique est terminée, le microprocesseur 21 règle la sortie R 5, s'arrête, puis attend un nouveau signal pour redémarrer de nouveau.

Les 24 bornes de sortie du microprocesseur 21 et ses deux bornes d'entrée sont indiquées avec leur fonction dans le tableau suivant :

| LIGNE (sorties) | FONCTION DE LA LIGNE |
|---|---|
| R0 | Prérègle le programmeur Z11 |
| R1 | Provoque la transmission d'impulsion venant de Q6 |
| R2 | Prérègle le basculeur de l'horloge |

| LIGNE (sorties) | FONCTION DE LA LIGNE |
|---|---|
| R3 | Voir RB |
| R4 | Provoque l'impulsion de sortie du stimulateur |
| R5 | Arrête le microprocesseur |
| R6 R7 | Sélectent l'entrée K4 et le flip-flop Z12 |
| R8 R9 | Règlent la sensibilité |
| RA | Assure une faible impédence d'entrée du stimulateur |
| RB | Déclenche le circuit de mesure de la résistance de la batterie |
| RC RD | Règlent la tension de sortie |
| RE | Déclenche le circuit d'essai de seuil |
| RF | Prérègle le flip-flop Z12 |
| 00 01 02 03 | Prérèglent les programmeurs |
| 04 05 06 07 | Règlent les tensions du circuit d'essai du seuil |
| (entrées) | |
| K4 | Signal soumis à essai |
| HLT | Arrête le fonctionnement du microprocesseur. |

Revendications de brevet.

1/ Procédé de commande et de réglage d'un appareil ou instrument alimenté électriquement notamment médical, plus spécialement un stimulateur cardiaque implantable, pourvu d'une batterie électrique, ce stimulateur pouvant travailler selon au moins un mode opératoire normal programmable et selon au moins un autre mode opératoire de commande, du fait de la présence d'un aimant placé au voisinage du stimulateur pour faire passer un commutateur de lecture à un état fermé, suivant la position et l'opération d'un programmeur extérieur pouvant programmer au moins un paramètre du stimulateur, caractérisé par le fait qu'on mesure la résistance interne de la batterie d'alimentation comme indication d'un prochain épuisement de celle-ci dans un premier mode de commande FMR, dans lequel on ramène également le rythme du stimulateur au rythme magnétique ; on fait recevoir par le stimulateur les instructions de commande à partir du programmeur extérieur dans un second mode de commande MAG successif ; on fait recevoir par le stimulateur de la part du programmeur externe, par voie télémétrique, la disposition de programmer au moins un paramètre du stimulateur, les données des mémoires du stimulateur étant modifiées en conséquence dans un troisième mode de commande PRG dans lequel le stimulateur passe automatiquement à partir du mode MAG si la commande est correcte ; on envoie en retour au programmeur extérieur des informations de contrôle sur les valeurs programmées dans un quatrième mode de commande TEL dans lequel le stimulateur passe à partir du mode PRG ou du second mode MAG pour revenir ensuite au mode MAG; on mesure le seuil de stimulation du patient dans un cinquième mode de commande TSL, dans lequel passe le stimulateur à partir du mode MAG en retirant l'aimant donc en ouvrant le commutateur de lecture ; on passe dans un sixième mode de commande VTL après le mode TSL et ensuite on passe, selon la programmation reçue, dans l'un des modes opératoires normaux, le dernier programmé.

2/ Procédé suivant la revendication 1, caractérisé par le fait que l'on fait travailler le stimulateur selon trois modes opératoires normaux programmables différents, notamment un premier mode VOO normal permanent, à rythme et tension de sortie constants programmés ; un second mode VVI dont la durée du cycle du stimulateur est divisée en trois sous-périodes, notamment une période réfractaire absolue pendant laquelle le stimulateur ne répond pas aux signaux de l'amplificateur de détection, une sous-période réfractaire relative pendant laquelle le stimulateur apprécie s'il convient ou non de passer dans la sous-période suivante, et une sous-période d'alerte dans laquelle le stimulateur répond à une impulsion détectée par l'amplificateur de détection en commençant un nouveau cycle sans générer d'impulsions de sortie ; et un troisième mode opératoire VVT différent du mode VVI par la réponse du stimulateur à l'amplificateur de détection pendant la sous-période d'alerte, pendant laquelle le stimulateur répond à une impulsion en arrêtant le cycle de fonctionnement après un retour de synchronisation puis en formant une impulsion de sortie.

3/ Dispositif de commande pour un appareil ou instrument alimenté électriquement notamment médical, plus spécialement un stimulateur cardiaque implantable, pourvu d'une batterie électrique, stimulateur pouvant travailler selon au moins un mode normal programmable et selon au moins un autre mode opératoire de commande du fait de la présence d'un aimant extérieur placé au voisinage du stimulateur pour faire passer un commutateur de lecture à un état fermé, en application du procédé selon l'une quelconque des revendications 1 et 2, caractérisé par le fait qu'il comporte un microprocesseur (21), un circuit interface (22) servant à relier le microprocesseur (21) au reste des circuits, des circuits analogiques (23 à 32) et un commutateur de lecture (34).

4/ Dispositif selon la revendication 3, caractérisé par le fait que les circuits analogiques (23 à 32) comportent un amplificateur de détection (23) un comparateur de détection (24), un émetteur-récepteur (25a), (25b) pourvu d'une antenne (33), un tripleur de tension (26) également sélecteur de courant de sortie, un régulateur de tension (27), un circuit de sortie (28), un circuit d'essai du seuil (29), un comparateur de résistance de la batterie (30), un limiteur de rythme et de largeur d'impulsion (31) et une horloge (32).

5/ Dispositif selon l'une quelconque des revendications 3 et 4, caractérisé par le fait que l'amplificateur (23) qui amplifie les signaux reçus et les transmet inversés au comparateur de détection (24), comporte un amplificateur (Z6) relié à un réseau de réaction constitué de résistances (R28, R29, R30) et de deux capacités (C16, C7) et à un réseau d'entrée constitué d'une résistance (R24) et d'une capacité (C4) ainsi que des résistances (R25, R26) une résistance (R27) et une capacité (C6).

6/ Dispositif selon l'une quelconque des revendications 3 et 4, caractérisé par le fait que le comparateur de détection (24) détectant si les impulsions en sortie de l'amplificateur de détection (23) sont positives ou négatives, comporte un amplificateur (27), un réseau de polarisation d'entrée constitué par les résistances (R31, R32, R34, R35) ainsi que les deux résistances (R8, R9) de couplage constitué par une capacité (C9) et une résistance (R43) et enfin une capacité (C10) et une résistance (R15), la sortie du comparateur (27) étant reliée à un déclencheur (Z13).

7/ Dispositif selon l'une quelconque des revendications 3 et 4, caractérisé par le fait que l'émetteur-récepteur comporte une partie émetteur (25a) comprenant un transistor (Q6), une capacité (C8), une résistance (R1) et une antenne commune (33) et une partie récepteur (25b) comprenant un transistor (Q4), une diode (D4) et deux résistances (R8, R10) ainsi qu'un déclencheur (Z13), le circuit du commutateur de lecture étant constitué par une résistance (R9) une capacité (C8), et le commutateur (34).

8/ Dispositif selon l'une quelconque des revendications 3, et 4 caractérisé par le fait que le tripleur de tension (26) recevant le courant de la batterie (35) à une certaine tension et fournissant aux autres circuits un courant à deux autres tensions préfixées, comprend une partie tripleur de tension (26a) et par une partie sélecteur du courant de sortie (26b) choisissant entre ces trois tensions possibles, la partie (26a) comportant un commutateur (Z.1), la partie X d'un commutateur (Z2) et deux capacités (C1) et (C2), la partie sélecteur de sortie comportant les parties Y et Z du commutateur (Z2).

9/ Dispositif selon l'une quelconque des revendications 3 et 4, caractérisé par le fait que le régulateur de tension (27) recevant le courant à tension maximale du tripleur de tension (26) et fournissant un courant d'alimentation de tous les circuits sur une ligne un potentiel Vss, se compose d'un transistor (Q2), trois résistances (R5, R6, R7) et un transistor (Q3).

10/ Dispositif selon l'une quelconque des revendications 3 et 4 caractérisé par le fait que le circuit de sortie (28) qui permet au circuit d'essai du seuil (29) de décharger une capacité de stockage (36) de l'énergie de sortie à des niveaux de tension préétablis et composé d'un commutateur (Z3) d'un transistor (Q1), de deux diodes (D6, D7) et de trois résistances (R2, R3) et (R44).

11/ Dispositif selon l'une quelconque des revendications 3 et 4, caractérisé par le fait que le circuit d'essai du seuil (29) est composé d'un comparateur (Z5) des résistances (R17), (R18), (R19), (R20), des résistances (R21), (R22), (R23) et (R16), ainsi que d'un transistor (Q5).

12/ Dispositif selon l'une quelconque des revendications 3 et 4, caractérisé par le fait que le comparateur de la résistance de la batterie (30) mesurant dans le mode FMR la résistance interne de la batterie (35) se compose d'un comparateur (Z4), de deux diodes (D5), (D8) et de quatre résistances (R11), (R12),(R13) et (R14).

13/ Dispositif selon l'une quelconque des revendications 4 et 5 caractérisé par le fait que le limiteur de rythme et de largeur d'impulsion (31), limitant le rythme de l'impulsion de sortie à une fréquence plus grande qu'une valeur préétablie et également la largeur de cette impulsion se compose d'un multivabrateur monostable non déclenchable (28), de deux capacités (C11) et (C12) et deux résistances réglables (R37) et (R38) ainsi qu'un déclencheur (Z13).

14/ Dispositif selon l'une quelconque des revendications 3 et 4, caractérisé par le fait que l'horloge (32) servant à régler les sous-programmes de transmission et de réception, se compose d'un multivibrateur astable (Z9), ainsi que d'une résistance (R39) et d'une capacité (C13) déterminant sa période.

15/ Dispositif selon l'une quelconque des revendications 3 et 4, caractérisé par le fait que le circuit interface (22), maintenant le microprocesseur (21) inactif jusqu'à ce qu'une opération logique soit nécessaire, comprend un rythmeur constitué par un compteur (Z11) comptant chaque front de montée de l'horloge (32) un multiplexeur (Z10), un flip-flop (Z12) et une porte "ET" (Z14).

16/ Dispositif selon l'une quelconque des revendications 3, 4 et 15, caractérisé par le fait que le circuit interface (22) comporte onze entrées à partir du microprocesseur (21) notamment (R2, R3, R5, R6, R0, 00, 01, 02, 03, RF) et six entrées de signalisation, notamment Q et OSC de l'horloge (32), (PP) du récepteur (25b), (RS) du commutateur de lecture (34) et (SA) du comparateur de détection (24 M) du comparateur de résistance ainsi que deux sortie (K4) et (HLT) vers le microprocesseur (21).

17/ Dispositif selon l'une quelconque des revendications 3, 4 et 16, caractérisé par le fait que le microprocesseur (21) comporte onze sorties vers l'interface (22), deux sorties (RC)´, (RD) vers le tripleur de tension (26), une sortie vers le comparateur de résistance de batterie (30), quatre sorties (04)´, (05),(06), (07) vers le circuit d'essai de seuil (29), une sortie (R4) vers les limiteur de rythme (31), une sortie(RE) vers les circuits de sortie (28) et d'essai de seuil (29), une sortie vers l'émetteur-récepteur (25a), (25b), une sortie (RA) vers le circuit de sortie (28) et deux sorties (R8), (R9) vers le comparateur de détection (24).

FIG.1

FIG.2

2/8

0064002

FIG.3

FIG.4

**FIG.5**

**FIG.6**

FIG.7

FIG.8

FIG.9

FIG.10

FIG.11

FIG.12

FIG.13

0064002